# EUROPEAN PATENT SPECIFICATION

(11) **EP 1 866 429 B1**
(45) Date of publication and mention of the grant of the patent: **19.01.2011**
(21) Application number: 06737552.7
(22) Date of filing: 09.03.2006
(51) Int. Cl.: C12N 5/071

(54) **METHODS OF ISLET ISOLATION AND TRANSPLANTATION**
VERFAHREN ZUR INSEL-ISOLIERUNG UND -TRANSPLANTATION
MÉTHODES D'ISOLATION ET DE TRANSPLANTATION D'ILOTS

(30) Priority: 14.03.2005 US 80797
(43) Date of publication of application: 19.12.2007
(73) Proprietor: Mediatech Inc., Herndon, VA 20171 (US)
(72) Inventor: DEOLDEN, James, Purcellville, Virginia 20132 (US); LAKEY, Jonathan, Edmonton, Alberta T6H 3Z8 (CA)
(74) Representative: Gowshall, Jonathan Vallance
(86) International application number: PCT/US2006/008390
(87) International publication number: WO 2006/099030

(56) References cited:
- EP-A- 0 440 925
- VOIGLIO ET AL: "Aerobic preservation of organs using a new perflubron /lecithin emulsion stabilized by molecular dowels" JOURNAL OF SURGICAL RESEARCH, ACADEMIC PRESS INC., SAN DIEGO, CA, US, vol. 63, no. 2, 1996, pages 439-446, XP002104382 ISSN: 0022-4804
- URUCHIHARA T ET AL: "A COMPARATIVE STUDY OF TWO-LAYER COLD STORAGE WITH PERFLUOROCHEMICAL ALONE AND UNIVERSITY OF WISCONSIN SOLUTION FOR RAT PANCREAS PRESERVATION" TRANSPLANTATION, WILLIAMS AND WILKINS, BALTIMORE, MD, US, vol. 57, no. 11, 15 June 1994 (1994-06-15), pages 1684-1686, XP001079705 ISSN: 0041-1337
- BRANDHORST D. ET AL.: 'Successful pancreas preservations by a perfluorocarbon-based one-layer method for subsequent pig islet isolation' TRANSPLANTATION vol. 79, 2005, pages 433 - 437, XP003009600
- TANIOKA Y. ET AL.: 'Augmentation of tissue ATP level during digestion using preoxygenated perfluorocarbon results in high islet yield-new strategy for islet isolation' TRANSPLANTATION PROCEEDINGS vol. 37, 2005, pages 220 - 222, XP004819906
- TSUJIMURA T. ET AL.: 'Influence of pancreas preservation on human islet isolation outcomes: impact of the two-layer method' TRANSPLANTATION vol. 79, 2004, pages 96 - 100, XP008095098

## Description

### FIELD OF THE INVENTION

The present disclosure relates to methods of isolating and transplanting islets, and more particularly relates to the use of a perfluorocarbon emulsification (ePFC) to enhance the viability of islets.

### BACKGROUND INFORMATION

An islet is a multi-cellular entity containing cells that produce insulin within the pancreas. The average person has about a million islets, and they contain approximately three percent of the total number of cells in the pancreas. The pancreas contains the islets of Langerhans, which house beta cells that produce insulin. The beta cells monitor glucose levels in the blood and release finely measured amounts of insulin to counterbalance glucose peaks. Type I and II diabetes develop when more than 90 percent of these beta cells are damaged.

The "Edmonton Protocol" transplants healthy islets into diabetic patients. Islet transplantation using the Edmonton Protocol is described in Shapiro, Ryan, and Lakey, Clinical Islet Transplantation - State of the Art, Transplantation Proceedings, 33, pp. 3502-3503 (2001); Ryan et al., Clinical Outcomes and Insulin Secretion After Islet Transplantation With the Edmonton Protocol, Diabetes, Vol. 50, April 2001, pp. 710-719; and Ryan et al., Continued Insulin Reserve Provides Long-Term Glycemic Control, Diabetes, Vol. 51, July 2002, pp. 2148-2157. Once in the liver, the cells develop a blood supply and begin producing insulin. The Edmonton Protocol may include 7-10 steps depending on the method employed. The first step involves the delivery of a specific enzyme (liberase) to a donor pancreas, which digests the pancreas tissue, but does not digest the islets. Following the digestion step, there are several successive steps for separating the islets from other cells in the pancreas. The separated islets are transplanted into the main vessel of the liver, known as the portal vein. The liver is able to regenerate itself when damaged, building new blood vessels and supporting tissue. Therefore, when islets are transplanted into the liver, it is believed that new blood vessels form to support the islets. The insulin that the cells produce is absorbed into the blood stream through these surrounding vessels and distributed through the body to control glucose levels in the blood.

Altogether, the steps of the Edmonton Protocol create a vigorous process that compromises the viability of islets, which have a fragile, three-dimensional structure and require large amounts of oxygen for growth and viability. During the process, islets may be damaged or destroyed due to non-optimal conditions of oxygen delivery, affecting the yield of healthy islets that are retrieved from a given donor pancreas. Furthermore, islet transplantation is severely limited by donor availability; frequently, two pancreata are required to obtain insulin independence in just one patient. As a result, there is a need for improved methods of isolation and transplantation that mitigate damage to islets and permit insulin independence from a single donor transplantation.

Improvements in the rate of single donor transplantation have been reported using the two layer method (TLM) of pancreas preservation; see, e.g., Salehi et al., Ameliorating ischemic injury during preservation and isolation of human islet cells using the two layer method with perfluorocarbon and University of Wisconsin solution, Transplantation 2005 (in press); Lakey et al., Human Pancreas Preservation Prior to Islet Isolation, Cell Preservation Technology, Vol. 1, No. 1, 2002, pp. 81-87; and Tsujimura et al., Human Islet Transplantation From Pancreases with Prolonged Cold Ischema Using Additional Preservation by the Two-Layer (UW Solution/ Perfluorochemical) Cold-Storage Method, Transplantation, Vol. 74, No. 12, Dec. 27, 2002, pp. 1687-1691. TLM involves the use of University of Wisconsin (UW) solution along with a perfluorocarbon (PFC) such as perfluorodecalin to preserve a human pancreas. UW has been employed in organ preservation for many years. It contains cell impermeant agents such as lactobionic acid that prevents cell swelling during cold storage, as well as glutathione, which works as an antioxidant, and adenosine, important for adenosine triphosphate synthesis. PFC, which is immiscible in water, has been helpful in pancreas preservation because of its high oxygen storage capability and low oxygen-binding constant, which allow it to store large amounts of oxygen for effective delivery to the ischemic organ. According to TLM methodology, the organ is preserved by immersing it in a container of the UW and PFC, where the organ is positioned to sit at the interface of the two liquids.

### SUMMARY OF THE INVENTION

The present invention as defined by the claims. There are disclosed methods for improving the viability and recovery of islets that are separated from a human donor organ for subsequent transplantation. In a preferred embodiment, the islets are separated from a donor pancreas and transplanted into the liver of a diabetic patient. The present invention includes a method of isolating pancreas islets including infusing at least one emulsified perfluorocarbon (ePFC) into an isolated donor pancreas and separating the pancreas islets from the isolated donor pancreas. The at least one ePFC is infused into the isolated donor pancreas prior to cell isolation and transplantation. The ePFC enhances the oxygenation of islets, thereby enhancing their health and viability so they may withstand a vigorous isolation procedure. The present invention not only preserves the donor organ using ePFC, but also rescues islets that would otherwise be damaged or destroyed during isolation and transplantation procedures. The separated islets may be injected into the portal vein of a liver where it is believed they develop a blood supply and assist in producing insulin and regulating blood glucose levels.

An object of the present invention is to provide a method of isolating islets comprising introducing at least one emulsified perfluorocarbon to a isolated donor organ, and separating islets from the donor organ.

Another object of the present disclosure is to provide a method of transplanting islets comprising introducing an emulsified perfluorocarbon to a donor organ, separating islets from the donor organ, and transplanting separated islets into a destination organ.

Another object of the present disclosure is to enhance the supply of oxygen to islets, thereby enhancing the health and viability of these cells so they may withstand the isolation and transplantation process.

Another object of the present disclosure is to decrease the number of islets that are damaged or destroyed during the isolation and transplantation process and increase the yield of viable, healthy, transplantable cells.

Another object of the present invention is to mitigate the need for multiple donor organs to achieve insulin independence.

Another object of the present invention is to allow donor organs to withstand a longer transit time.

Another object of the present invention is to rescue donor organs that would otherwise be considered unsuitable for use.

Another object of the present invention is to standardize isolation procedures that are used for donor organs of varying quality.

These and other aspects of the present invention will become more readily apparent from the following detailed description and appended claims.

### TABLES

Table 1 presents islet enumeration, size, and viability for experiments conducted on rat pancreata.
Table 2 presents tissue ATP levels over time for an experiment conducted using human donor pancreata..
Table 3 summarizes Tissue Energy Charge over time for an experiment conducted using human donor pancreata.

### FIGURES

Figure 1 is a box plot of non-cotrected islet enumeration for an experiment conducted on rat pancreata.
Figure 2 is a box plot of corrected islet enumeration for an experiment conducted on rat pancreata.
Figure 3 presents photographs showing differential fluorescence staining from an experiment conducted on rat pancreata.

### DETAILED DESCRIPTION

The present invention provides methods for improving the viability and recovery of islets that are separated from a isolated donor organ for subsequent transplantation. In a preferred embodiment, the islets are separated from a donor pancreas and transplanted into the liver of a diabetic patient. While the description contained herein primarily refers to cell transplantations into livers, it is to be understood that the invention may be utilized for other transplant destinations, such as the testes. As used herein, the terns patient, donors, and donees refer to humans and members of the animal kingdom.

The present invention includes one or more emulsified perfluorocarbons (ePFCs) for introduction into a donor pancreas prior to cell isolation and transplantation. This introduction may be accomplished by infusion into the donor pancreas, which also encompasses injection or ePFC into the donor pancrease. The ePFC enhances the oxygenation of islets, thereby enhancing their health and viability so they may withstand a vigorous isolation procedure such as the Edmonton Protocol. The present invention not only preserves the donor organ using ePFC, but also rescues islets that would otherwise be damaged or destroyed by the isolation and transplantation procedure.

The ePFC may comprise an injectable emulsification of water and any suitable PFC, for example, perfluorodecalin or perfluoroctylbromide. PHER-O2 is an example of a commercially available ePFC produced by the Sanguine Corporation. Although the PFC micelles in the emulsification are not limited to any particular size, the average size (i.e., width) of each micelle preferably ranges from about 275 to 310 microns; the most preferred micelle size is about 290 microns. The emulsification may appear cloudy and opaque. While the amount of PFC in the emulsification may vary, it preferably ranges from about 40 to 90 percent by weight. For a typical donor pancreas, anywhere from 20 to 150 milliliters of the emulsification may be used to enhance the viability of islets prior to isolation. The emulsification may be slowly infused in small portions to avoid distension or rupture of the pancreatic duct. However, the amount of PFC and the total amount of emulsification, as well as the infusion method employed, may vary considerably and/or depart from the values stated above depending on the quality, health, and size of the donor organ, the time at which it was removed from the donor, and the method of transporting the organ.

After the ePFC is prepared and bottled, it is oxygenated for a period of time using sterile oxygen. The emulsification is then infused into the pancreatic duct of the donor pancreas using a thin needle, canula, plastic tube, or similar device. Prior to the first step of the islet isolation procedure (enzymatic digestion), the emulsification may be left intact for approximately four hours to allow for adequate oxygenation. Infusion is preferable to immersing the pancreas in a solution because in a micellar state, the PFC has a greater surface area and therefore, makes contact with a greater number of islets. A TLM solution of PFC and UW, on the other hand, is not capable of being injected into an organ. Once infused, the ePFC releases oxygen to the islets contained within the pancreas. The release of oxygen from PFC is well documented as there have been many attempts to use PFC as an artificial blood.

Following infusion of ePFC, the Edmonton Protocol or another suitable procedure may be applied to separate the islets from other cells in the donor pancreas. The Edmonton Protocol involves multiple steps, including distention of the pancreas through ductal perfusion, followed by enzymatic and mechanical digestion, and purification of islets using density gradient centrifugation. The enzymatic digestion step is a vigorous process that typically damages or destroys many islets, leading to a low yield of viable, transplantable, post-isolation cells. However, with the infusion of ePFC, more oxygen is available for the islets to thrive, and therefore, a greater number of these cells survive the enzyme destruction and other mechanical steps of the process.

An islet, like an organelle, has a distinctive shape and function, and contains more than one type of cell (e.g., the beta cell) within the islet unit. There are many parameters that can be used to determine the goodness (i.e., health and vitality) of a specific cell. Two measures of viability are 1) determination of ATP levels, and 2) measurement of Tissue Energy Charge.

During a typical isolation procedure using the Edmonton Protocol, ATP levels decline. However, when ePFC is infused into a donor pancreas, ATP levels may actually stay the same or increase following isolation procedures Following ePFC infusion, the ATP levels may increase many times compared to an organ that is not infused with ePFC. The rise in ATP suggests that the ePFC may enhance or rescue the goodness of the pancreas cells, allowing them to withstand the vigorous isolation procedure. The rise in AIP also suggests that the ePFC may increase the yield of viable, healthy, transplantable cells that are separated from the pancreas. Certain modes of death may have a deleterious effect on the goodness of the cells in the pancreas, and consequently, certain donor organs may be considered unsuitable for use. The rise in ATP, however, suggests that ePFC may be used to rescue donor organs that would otherwise be rejected due to mode of death. The rise in ATP also suggests that the addition of ePFC may allow a donor organ to be in transit longer without significantly compromising the viability of the cells contained within. Like ATP, Tissue Energy Charge tends to decrease in the absence of ePFC, and stay the same or increase with the addition of ePFC. The details of experiments conducted on rat pancreata and human donor organs are provided below.

### EXAMPLES

### Example 1

An experiment was conducted to compare the effectiveness of UW, TLM, and ePFC in their ability to maintain rat pancreata for subsequent isolation. Six rat pancreata were assembled into three groups. Group 1 was immersed in a solution of UW, Group 2 was immersed using TLM at the interface of UW and PFC, and Group 3 was infused with ePFC. Hank's Balanced Salt Solution (HBSS) was used for distention of the pancreata because ePFC did not achieve adequate distention.

Two pancreata were transferred into 1 of 3 different solutions in 500mL polypropylene straight-side wide-mouth jars. Group 1 consisted of 60mL UW solution, Group 2 of the two layer solution containing 60mL of UW solution ("Viaspan") and 60mL of PFC (pre-oxygenated for 30 minutes at 100% oxygen), and Group 3 of 60mL ePFC (pre-oxygenated for 30 minutes at 100% oxygen). Both the employed immiscible PFC and ePFC contained perfluorodecalin, a type of PFC. In all three groups, metal grills were applied above the pancreata to prevent floating, especially in Group 2, where the pancreata were positioned to sit at the interface between UW and PFC. All three containers were immediately placed on ice and into cold storage (~4°C) for 18 hours.

After 18 hours of cold storage, pancreata were removed from cold storage and islets were isolated using standard techniques. Drawn islets were placed into tubes containing approximately 25ml HBSS+, and were centrifuged for 2 minutes at 500 x g and then washed with HBSS+ and placed into M199 solution for one hour at 37°C.

Randomized samples were taken (100µl or 1:50), and placed into 7 drops dithizone for staining. After 5 to 10 minutes, samples were smeared onto Petri dishes and counted using a grid system under microscopy by two blinded enumerators. This grid system uses multiplication factors to give an islet equivalent (IE) count for islets at 150µm in diameter.

Islet viability was quantified via membrane integrity differential staining. A sample was taken and combined with 10µL 5mM SYTO Green 13 in dimethyl sulfoxide. After 2 minutes, 5µL of 25.4mM ethidium bromide in Dulbecco's Phosphate Balanced Salt (DPBS) solution was added. Slides were streaked and viability determined by counting viable and non-viable islets under fluorescence microscopy. Pictures were taken of each group.

Three 1:50 samples from each group (2 pancreata per group) were placed in low glucose (2.8mM) solution for 2 hours at 37°C and 95%CO₂, followed by removal of supernatant, which was frozen for later testing. Islets samples were then placed into high glucose (20mM) solution for 2 hours at 37°C and 95% CO₂. Again, supernatant was removed and frozen. Samples were analyzed for insulin content by ELISA. It should be noted that this procedure was discontinued as it did not yield significant results, especially taking into account the cost of testing samples.

Islet number, average size and viability were compared between groups using ANOVA testing on SPSS 11.5 software. If ANOVA revealed significance at p=0.05, Bonferroni post hoc analysis was used to locate significantly different groups. Table 1 summarizes the results, which are expressed as mean±SEM. These results show an increase in islet size and viability for Group 3 compared to Groups 1 and 2. However, because of the small size of the rat pancreata and the difficulty associated with injecting the ePFC into the small ducts of these tiny organs, it was determined that further experiments using human pancreata were required.

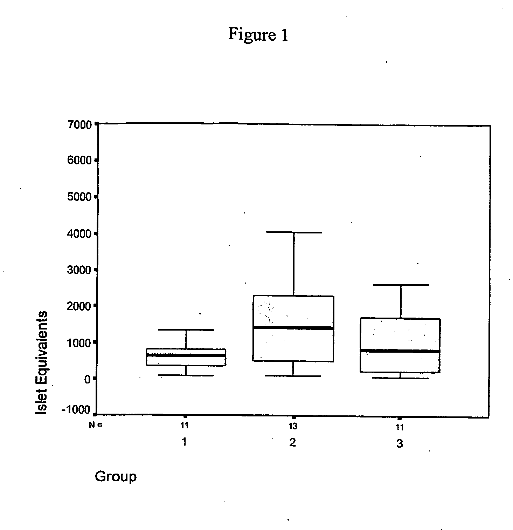

Figure 1 presents a box plot of non-corrected islet enumeration (IE) for each group. All of the groups, particularly Group 3, were negatively skewed. There were a few major outliers initially in the IE counts. A low outlier was removed from Group 2 and two from Group 3 because the counts were extremely low (over three times lower than the next lowest value), presumably due to problematic staining. Furthermore, a count from Group 2 and 3 was removed because they were extremely high, due to the presence of a small number of extremely large islets which skewed the count (each was roughly three or more standard deviations from the corrected means).

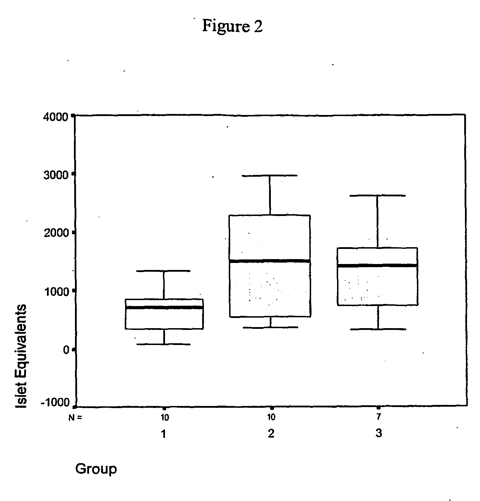

Figure 2 presents a box plot of corrected islet enumeration, showing a more normal distribution after removal of the outliers. ANOVA analysis of the corrected data set revealed a significant difference (p=0.041). Bonferonni post hoc analysis revealed significance between Group 2 (1491±280 IE) and Group 1 (667±118 IE) (p=0.048). (Note: For Group 2, enumeration is expressed per two rat pancreata; i.e., two pancreata per trial.) Group 2 and 3 (1328+301 IE) closely paralleled each other (p=1.00), although there was no significance between Group 3 and 1. Some samples had long filamentous tissue damage under microscopy that caused tissue aggregation, probably from excessive collagenase digestion. However, no group showed an elevated propensity for displaying such damage.

The IE system uses grids to give estimates of islet size, which can then be employed to determine average size for each group per trial. There was no significant difference (p=0.43) in mean islet diameter between Group 1 (120±6.5 µm), Group 2 (120±5 µm), and Group 3 (133±12 µm), although Group 3 was slightly larger than the other two.

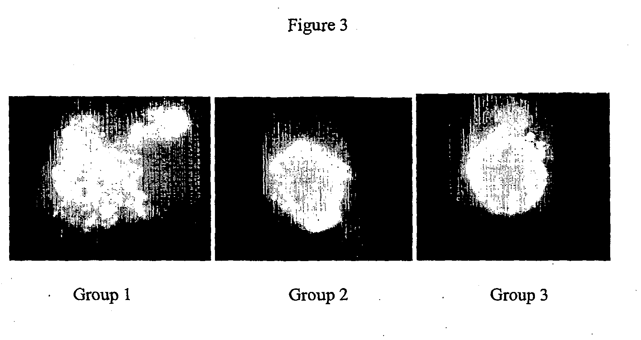

SYTO/EB membrane integrity staining provided both quantitative and qualitative assessment of islet viability. Figure 3 presents photographs of differential fluorescent staining on Groups 1, 2, and 3. There was no significant difference in percent viability (p=0.58) between Group 1 (52±9%), Group 2 (61±9%), and Group 3 (65±12%). In all groups, there was fragmentation along the border of some islets, often contributing to their qualification as non-viable; there was also some tissue aggregation in all groups. Group 1 was furthermore characterized by central islet death. Group 2 had viable islets with intact borders; death was generally due to fragmentation around the islet border. Viable tissue in Group 3 was notably spheroidal; non-viable islets in Group 3 were generally characterized by border fragmentation and infrequently by central death.

As mentioned above, static incubation was discontinued in this experiment. Static incubation (SI) is the ratio of insulin production in high glucose solution to that in low glucose solution. While the insulin stimulation index in response to glucose was expected to be greater than one, in all cases, it was less than one. For instance, a third trial revealed a mean SI of 0.59 for Group 1, 0.62 for Group 2, and 0.39 for Group 3.

This experiment revealed a significant improvement in islet yield after 18 hour preservation using the two layer method over UW, as shown in Table 1. The IE post-preservation closely paralleled between ePFC and the two layer method ((p=1.00) from Bonferonni's post hoc analysis), with the two layer method producing a slightly higher mean value (1491 IE versus 1328 IE). However, a few significant outliers had to be removed from the data set. Islet isolation is subject to countless variables affecting its outcome, such as in collagenase activity, donors, mechanical digestion, and islet purification. Furthermore, problems with islet staining contributed to outliers in the counts. Dithizone, the stain used for enumeration, chelates zinc, the latter which normally forms a hexamer with insulin. Thus the observed deficiency in insulin production in preserved islets (as seen by static incubation assay) probably contributed to the problematic staining.

Static incubation revealed a deficiency in all preserved islets' ability to produce insulin when subjected to hyperglycemic conditions. There are a few explanations for this occurrence. Firstly, the β-cells may have had decreased insulin content due to cold storage. Presumably, the low temperature storage inhibited normal cell metabolic processes, thus preventing production of insulin for storage, as well as expression of insulin-producing enzymes. Culture of islets in temperatures below 37°C has been implicated in the degranulation and impaired insulin response of islet β cells, and this is likely the same for pancreas cold preservation. Secondly, the isolation procedure is harsh on islets (digestion and density gradient centrifugation), and may have caused degranulation and/or impaired insulin production. A third possibility is that most islets were not viable. However, the notion that the islets were merely non-viable is non-concordant with the results of the differential membrane integrity staining. In all cases, viability derived by this staining procedure was reasonably high (all means were above 50%), considering the length of cold ischemia time (18 hours). It is therefore more likely that the static incubation assay displayed low SI values because of the combined effects of low temperature storage and the perturbations associated with islet isolation.

Generally, islets become centrally necrotic in low oxygen conditions. It is reasonable to estimate that larger islets are more likely subject to central necrosis in ischemic conditions, due to decreased islet surface area to diameter ratio, and thus decreased oxygen diffusion to the central tissue. Although islet size was not significantly different between groups, it was slightly higher in the ePFC group compared to the other groups; this may imply better oxygenation in the ePFC group.

There was no significant difference in the percent viability (derived from membrane integrity data) between the three groups, although mean percent viability was slightly higher in Group 3 (65%) than Group 2 (61%) or 1 (52%). The islets in the UW group appeared to have greater central necrotic damage than the other two groups, most likely due to oxygen restraints. TLM seemed to best preserve islets from central death, although mean percent viability was slightly lower than the ePFC group. In this group and the ePFC group, most non-viable islets were as such because of fragmentation; presumably, the isolation procedure disrupted the border of these islets, which were already fragile from cold storage.

It appears from enumeration data that TLM was most effective at preserving islets in whole pancreas preservation. However, the mean number of islets in the ePFC group closely paralleled the TLM group (p=1.00), and percent viability and islet size were slightly higher, although there was no statistical significance in either case. It is likely that some of the perceived benefit of TLM over ePFC is due to the absence of cell impermeant agents, glutathione, and adenosine, all of which are found in UW.

This experiment confirms that the two layer method is more effective than UW at preserving pancreata for islet isolation, as noted previously. Furthermore, a novel ePFC may also prove beneficial in maintaining normal islet physiology for transplantation. Emulsified PFC has a few possible benefits over immiscible PFC. For instance, it may be possible to perfuse the emulsion into the pancreas upon its recovery from a donor, thus allowing more direct oxygenation of tissues within the organ and increasing the effective surface area of oxygen delivery. The attempt at perfusing ePFC solution into the rat pancreas was unsuccessful, as ample distention was not achieved. It is likely that this was due to the high viscosity of the solution and the small size of the rat pancreatic duct system. Perfusion may not be problematic in the larger human pancreas. Moreover, dilution of ePFC may make future attempts at pancreas distention with this solution more successful.

### Example 2

Human donor pancreata were obtained to test the effect of ePFC on cellular viability. Two donor pancreata, referred to as Donors 1 and 2, were used as controls and did not receive ePFC infusion. Two experimental donor pancreata, referred to as Donors 3 and 4, were infused with ePFC. The islets were isolated using the standard Edmonton protocol.

Table 2 presents tissue ATP levels pre- and post-purifcation for Donors 1-4. Table 3 presents Tissue Energy Charge pre- and post-purification for Donors 1-4. "Purification" refers to the purification of islets that is conducted as part of the Edmonton protocol. The experimental data for Donors 3 and 4 shows markedly increased ATP and Tissue Energy Charge compared to the control data for Donors 1 and 2. In fact, the controls show reductions in most of these energy levels. The increase in ATP and Tissue Energy Charge clearly demonstrates that the viability and health of the islets treated with ePFC is enhanced and that these islets have been revived or rescued.

Whereas particular embodiments of this invention have been described above for purposes of illustration, it will be evident to those skilled in the art that numerous variations of the details of' the present invention may be made without departing from the invention as defined in the appended claims.

**Table 1**

| Group | Islet Enumeration (IE) | Islet Size (µm) | Number of counts†: Islet enumeration and size (N) | Viability (%) |
|---|---|---|---|---|
| 1 | 667±118* | 120±6.5 | 10 | 52±9 |
| 2 | 1491±280* | 120±5 | 10 | 61±9 |
| 3 | 1328±301 | 133±12 | 7 | 65±12 |

| | | | | |
|---|---|---|---|---|
| * Significant difference at p<0.05 (Bonferonni's post hoc analysis) † Number of counts are those after removal of outliers Note: Data are expressed as mean ± SEM. | | | | |

**Table 2**

| **Tissue ATP Level (nmol/mg protein)** | | |
|---|---|---|
| **Donor** | **Pre-purification** | **Post-purification** |
| **1 (Control)** | 0.371 | 0.981 (After 12 hr) |
| | | 0.851 (After 24 hr) |
| **2 (Control)** | 4.047 | 1.723 (After 9 hr) |
| **3 (Experimental)** | 0.652 | 7.042 (After 4 hr) |
| **4 (Experimental)** | 0.354 | 5.011 (After 6 hr) |

**Table 3**

| **Tissue Energy Charge** | | |
|---|---|---|
| **Donor** | **Pre-Purification** | **Post-Purification** |
| **1 (Control)** | 0.36 | 0.24 (After 12 hr) |
| | | 0.19 (After 24 hr) |
| **2 (Control)** | 0.42 | 0.34 (Aftet 9 hr) |
| **3 (Experimental)** | 0.57 | 0.63 (After 4 hr) |
| **4 (Experimental)** | 0.49 | 0.58 (After 6 hr) |

## Claims

1. A method of isolating pancreas islets comprising:
infusing at least one emulsified perfluorocarbon into an isolated donor pancreas, and
separating the pancreas islets from the isolated donor pancreas.

2. The method of Claim 1, wherein the isolated donor pancreas comprises a human pancreas.

3. The method of Claim 1, wherein the at least one emulsified perfluorocarbon is selected from the group comprising perfluorodecalin and perfluoroctylbromide.

4. The method of Claim 1, wherein the at least one emulsified perfluorocarbon comprises from 40 to 90 percent by weight perfluorocarbon.

5. The method of Claim 1, wherein the at least one emulsified perfluorocarbon comprises a plurality of micelles.

6. The method of Claim 5, wherein each micelle in the plurality of micelles has an average size ranging from 275 to 310 microns.

7. The method of Claim 6, wherein each micelle in the plurality of micelles has an average size of 290 microns.

8. The method of Claim 1, wherein 20 to 150 millimeters of the at least one emulsified perfluorocarbon is infused into the isolated donor pancreas.

9. The method of Claim 1, wherein the at least one emulsified perfluorocarbon is infused into the isolated donor pancreas by infusion into a pancreatic duct.

10. The method of Claim 1, wherein the islets are separated from the isolated donor pancreas according to the Edmonton Protocol.

11. The method of Claim 1, wherein ATP levels increase following infusion of the at least one emulsified perfluorocarbon.

12. The method of Claim 1, wherein ATP levels stay about the same following infusion of the at least one emulsified perfluorocarbon.

13. The method of Claim 1, wherein Tissue Energy Charge increases following infusion of the at least one emulsified perfluorocarbon.

14. The method of Claim 1, wherein Tissue Energy Charge stays about the same following infusion of the at least one emulsified perfluorocarbon.

15. The method of Claim 1, wherein the at least one emulsified perfluorocarbon rescues the isolated donor pancreas that would otherwise be considered unsuitable for use.

16. An emulsified perfluorocarbon for use in a method of transplanting pancreas islets wherein:
pancreas islets are to be isolated by a method according to any preceding claim, and
the separated pancreas islets are to be transplanted into a destination organ.

17. The emulsified perfluorocarbon of Claim 16, wherein the destination organ comprises a liver of a diabetic patient.

## Patentansprüche

1. Verfahren zum Isolieren von Pankreasinseln, das Folgendes umfasst:
Infundieren mindestens eines emulgierten Perfluorkohlenstoffs in eine isolierte Spenderpankreas und
Trennen der Pankreasinseln von der isolierten Spenderpankreas.

2. Verfahren nach Anspruch 1, wobei die isolierte Spenderpankreas eine menschliche Pankreas umfasst.

3. Verfahren nach Anspruch 1, wobei der mindestens eine emulgierte Perfluorkohlenstoff aus der Gruppe umfassend Perfluordecalin und Perfluoroctylbromid ausgewählt ist.

4. Verfahren nach Anspruch 1, wobei der mindestens eine emulgierte Perfluorkohlenstoff von 40 bis 90 Gewichtsprozent Perfluorkohlenstoff umfasst.

5. Verfahren nach Anspruch 1, wobei der mindestens eine emulgierte Perfluorkohlenstoff mehrere Mizellen umfasst.

6. Verfahren nach Anspruch 5, wobei jede Mizelle der mehreren Mizellen eine durchschnittliche Größe aufweist, die von 275 bis 310 Mikron reicht.

7. Verfahren nach Anspruch 6, wobei jede Mizelle der mehreren Mizellen eine durchschnittliche Größe von 290 Mikron aufweist.

8. Verfahren nach Anspruch 1, wobei 20 bis 150 Millimeter des mindestens einen emulgierten Perfluorkohlenstoffs in die isolierte Spenderpankreas infundiert werden.

9. Verfahren nach Anspruch 1, wobei der mindestens eine emulgierte Perfluorkohlenstoff mittels Infusion in einen Pankreasgang in die isolierte Spenderpankreas infundiert wird.

10. Verfahren nach Anspruch 1, wobei die Inseln gemäß dem Edmonton-Protokol von der isolierten Spenderpankreas getrennt werden.

11. Verfahren nach Anspruch 1, wobei die ATP-Spiegel nach Infusion des mindestens einen emulgierten Perfluorkohlenstoffs steigen.

12. Verfahren nach Anspruch 1, wobei die ATP-Spiegel nach Infusion des mindestens einen emulgierten Perfluorkohlenstoffs ungefähr gleich bleiben.

13. Verfahren nach Anspruch 1, wobei die Energieladung des Gewebes nach Infusion des mindestens einen emulgierten Perfluorkohlenstoffs steigt.

14. Verfahren nach Anspruch 1, wobei die Energieladung des Gewebes nach Infusion des mindestens einen emulgierten Perfluorkohlenstoffs ungefähr gleich bleibt.

15. Verfahren nach Anspruch 1, wobei der mindestens eine emulgierte Perfluorkohlenstoff die isolierte Spenderpankreas rettet, die andernfalls als zur Verwendung ungeeignet erachtet werden würde.

16. Emulgierter Perfluorkohlenstoff zur Verwendung in einem Verfahren zum Transplantieren von Pankreasinseln, wobei:
Pankreasinseln mit einem Verfahren nach einem der vorhergehenden Ansprüche isoliert werden sollen und
die getrennten Pankreasinseln in ein Zielorgan transplantiert werden sollen.

17. Emulgierter Perfluorkohlenstoff nach Anspruch 16, wobei das Zielorgan eine Leber eines diabetischen Patienten umfasst.

## Revendications

1. une méthode d'isolation des îlots pancréatiques comprenant :
l'infusion d'au moins un perfluorocarbone émulsifié dans un pancréas isolé chez un donneur, et
la séparation des îlots pancréatiques du pancréas isolé chez un donneur.

2. La méthode de la revendication 1, dans laquelle le pancréas isolé chez un donneur comprend un pancréas humain.

3. La méthode de la revendication 1, dans laquelle le perfluorocarbone minimal émulsifié est choisi dans le groupe comprenant le perfluorodecaline et le perfluorooctylbromide.

4. La méthode de la revendication 1, dans laquelle le perfluorocarbone minimal émulsifié comprend de 40 à 90 pourcent du poids en perfluorocarbone.

5. La méthode de la revendication 1, dans laquelle le perfluorocarbone minimal émulsifié comprend une pluralité de micelles.

6. La méthode de la revendication 5, dans laquelle chaque micelle de la pluralité de micelles a une taille moyenne allant de 275 à 310 microns.

7. La méthode de la revendication 6, dans laquelle chaque micelle de la pluralité de micelles a une taille moyenne de 290 microns.

8. La méthode de la revendication 1, dans laquelle 20 à 150 millimètres du perfluorocarbone minimal émulsifié est infusé dans le pancréas isolé chez un donneur.

9. La méthode de la revendication 1, dans laquelle le perfluorocarbone minimal émulsifié est infusé dans le pancréas isolé chez un donneur par infusion dans la canal de Wirsung.

10. La méthode de la revendication 1, dans laquelle les îlots sont séparés du pancréas isolé chez un donneur selon le protocole d'Edmonton.

11. La méthode de la revendication 1, dans laquelle les taux d'ATP augmentent après l'infusion du perfluorocarbone minimal émulsifié.

12. La méthode de la revendication 1, dans laquelle les taux d'ATP restent environ les mêmes après l'infusion du perfluorocarbone minimal émulsifié.

13. La méthode de la revendication 1, dans laquelle la charge énergétique des tissus augmente après l'infusion du perfluorocarbone minimal émulsifié.

14. La méthode de la revendication 1, dans laquelle la charge énergétique des tissus reste environ la même après l'infusion du perfluorocarbone minimal émulsifié.

15. La méthode de la revendication 1, dans laquelle le perfluorocarbone minimal émulsifié récupère le pancréas isolé chez un donneur qui serait sinon considéré comme inapproprié à l'utilisation.

16. Un perfluorocarbone émulsifié pour l'utilisation dans une méthode de greffe d'îlots pancréatiques dans laquelle :
les îlots pancréatiques doivent être isolés selon une méthode conforme à l'une quelconque des revendications précédentes, et les îlots pancréatiques séparés doivent être greffés dans un organe de destination.

17. Le perfluorocarbone émulsifié de la revendication 16, dans laquelle l'organe de destination comprend un foie chez un patient diabétique.
